# EUROPEAN PATENT APPLICATION

(11) **EP 4 632 761 A1**
(43) Date of publication of application: **15.10.2025**
(21) Application number: 23900926.9
(22) Date of filing: 10.11.2023
(51) Int. Cl.: G16H 40/60, G16H 30/20, G16H 50/20, A61B 8/00, A61B 8/08, A61B 5/15

(54) **MEDICAL SYSTEM CONTROL METHOD, COMPUTER PROGRAM, AND APPARATUS**

(30) Priority: 07.12.2022 KR 20220169995
(71) Applicant: Airs Medical Inc., Seoul 08788 (KR)
(72) Inventor: CHOI, Yeongjae, Seoul 06562 (KR)
(74) Representative: Isarpatent
(86) International application number: PCT/KR2023/018089
(87) International publication number: WO 2024/122907

(57) **Abstract**

A medical system control method that is performed by a computing device including at least one processor according to one embodiment of the present invention includes: obtaining multi-modal data from a medical system that operates according to set control conditions; generating condition change data for changing the control conditions of the medical system based on the multi-modal data by using a first artificial intelligence model; and controlling the medical system based on the condition change data.

## Description

### Technical Field

The present disclosure relates to a medical system control method, computer program and apparatus, and more particularly, to a medical system control method, computer program and apparatus using multi-modal data and an artificial intelligence model.

### Background Art

Medical systems include various types of medical equipment and various medical devices, and various types of medical data are generated from individual devices. For example, a medical imaging device generates two-dimensional or three-dimensional images.

Alternatively, when medical equipment performs an operation of assisting in a medical procedure, the movement of the medical equipment and a series of operations performed by the medical equipment are generated as data. In this case, subjective data, such as responses of a patient who is a subject, is also generated.

Such medical data generated in medical systems is input to an artificial intelligence model and serves to detect a lesion or operate medical equipment.

More specifically, artificial intelligence models trained based on medical images may identify skin conditions and symptoms in the field of dermatology, or may find nodules or tumors in CT or X-ray images in the field of radiology. Alternatively, in the field of pathology, they may identify the locations of tumors in tissue slides.

In another field, artificial intelligence models are trained on actual surgical processes of doctors through trial & error, and thus, allow doctors to remotely control robots. Alternatively, they may be trained to utilize robots for repetitive tasks such as suturing or knot-tying.

General artificial intelligence models are frequently specialized for specific types of data such as images. However, in actual medical environments, multiple types of data are usually mixed together, so that models that consider data of various dimensions are required. Furthermore, when complex data is used, there may be differences in suitability between individual types of data. Since the reliability of medical operations may be affected by these differences in suitability, a process of determining the suitability of data needs to be performed additionally for reliable medical operations.

### Disclosure

### Technical Problem

The present disclosure is intended to overcome the problems of the above-described conventional art, and is directed to a medical system control method, computer program and apparatus that change control conditions of a medical system based on multi-modal data of various dimensions generated in the medical system.

However, objects to be achieved by the embodiments are not limited to the above-described object, and other objects may be present.

### Technical Solution

According to one embodiment of the present disclosure for achieving the above-described object, there is disclosed a medical system control method that is performed by a computing device. The medical system control method includes: obtaining multi-modal data from a medical system that operates according to set control conditions; generating condition change data for changing the control conditions of the medical system based on the multi-modal data by using a first artificial intelligence model; and controlling the medical system based on the condition change data.

Alternatively, the generating includes: calculating the suitability of the multi-modal data; and generating the condition change data when the suitability is lower than a reference value.

Alternatively, the medical system control method further includes: performing a preset medical operation by using the medical system when the suitability is equal to or higher than the reference value.

Alternatively, the performing a preset medical operation includes: calculating the reliability of the medical operation based on the multi-modal data by using a second artificial intelligence model; performing the medical operation when the reliability is equal to or higher than a reference value; and generating the condition change data when the reliability is lower than the reference value.

Alternatively, the medical system includes an ultrasound probe, and the multi-modal data includes an ultrasound image.

Alternatively, the condition change data changes at least one of the position of the ultrasound probe and the contact pressure between the ultrasound probe and the body.

Alternatively, the second artificial intelligence model is a model trained to infer characteristics of a blood vessel area of the ultrasound image based on the contact pressure between the ultrasound probe and the body included in the multi-modal data.

Alternatively, the medical system includes a venipuncture device, and the multi-modal data includes an infrared image or an ultrasound image.

Alternatively, the generating includes generating the condition change data to change at least one of the degree of body compression of a body compression unit included in the venipuncture device and the position of the venipuncture device.

According to one embodiment of the present disclosure for achieving the above-described object, there is disclosed a medical system control apparatus. The medical system control apparatus includes: memory configured to store multi-modal data obtained from a medical system; and a processor configured to calculate the suitability of the multi-modal data, to generate condition change data for changing control conditions of the medical system based on the multi-modal data by using a first artificial intelligence model when the suitability is lower than a reference value, and to perform a preset medical operation by using the medical system when the suitability is equal to or higher than the reference value.

Alternatively, the medical system includes a medical image acquisition device or a venipuncture device.

Alternatively, the multi-modal data includes a medical image, and further includes at least one of the time at which the medical image is acquired, a sensing value obtained by a sensor included in the medical image acquisition device or the venipuncture device, an encoder value of a motor included in the medical image acquisition device or the venipuncture device, and the position, displacement, and operation time of an ultrasound probe included in the medical image acquisition device or the venipuncture device.

According to one embodiment of the present disclosure for achieving the above-described object, there is disclosed a computer program that is stored in a computer-readable storage medium. The computer program causes operations for controlling a medical system to be performed when executed by at least one processor. In this case, the operations include operations of: obtaining multi-modal data from a medical system that operates according to set control conditions; calculating the suitability of the multi-modal data; and generating condition change data for changing the control conditions of the medical system based on the multi-modal data by using a first artificial intelligence model when the suitability is lower than a reference value, and performing a preset medical operation by using the medical system when the suitability is equal to or higher than the reference value.

### Advantageous Effects

According to the technical solution of the present disclosure described above, the present disclosure may improve the accuracy of inference by inputting data of various dimensions generated from a medical system as well as a medical image to an artificial intelligence model.

In addition, according to the technical solution of the present disclosure described above, the present disclosure allows the operation of the medical system to be performed only when multi-modal data having a predetermined level of suitability is obtained, so that the precision of the medical system can be improved.

### Description of Drawings

FIG. 1 is a block diagram of a computing device according to one embodiment of the present disclosure;
FIG. 2 is a block diagram of a medical system control apparatus according to one embodiment of the present disclosure;
FIG. 3 is a flowchart showing a medical system control method according to one embodiment of the present disclosure;
FIG. 4 is a flowchart showing a method of training the first artificial intelligence model according to one embodiment of the present disclosure; and
FIG. 5 is a flowchart showing a medical system control method to which condition control is applied according to one embodiment of the present disclosure.

### Mode for Invention

Embodiments of the present disclosure will be described in detail below with reference to the accompanying drawings so that those having ordinary skill in the art of the present disclosure (hereinafter, those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The term "image" used herein may refer to multidimensional data composed of discrete image elements. In other words, "image" may be understood as a term referring to a digital representation of an object that can be seen by the human eye. For example, "image" may refer to multidimensional data composed of elements corresponding to pixels in a two-dimensional image. "Image" may refer to multidimensional data composed of elements corresponding to voxels in a three-dimensional image.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

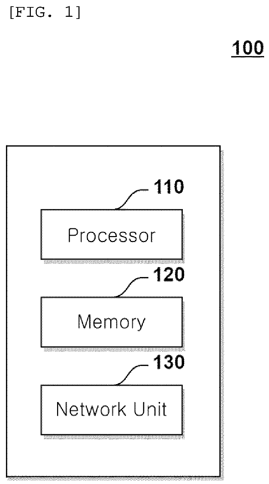

FIG. 1 is a block diagram of a computing device according to one embodiment of the present disclosure.

A computing device 100 according to one embodiment of the present disclosure may be a hardware device or a part of a hardware device that performs the comprehensive processing and computation of data, or may be a software-based computing environment connected over a communication network. For example, the computing device 100 may be a server that is a main agent for performing an intensive data processing function as a computing device and sharing resources, or may be a client that shares resources through interaction with a server. Alternatively, the computing device 100 may be a cloud system in which multiple servers and clients comprehensively process data while interacting with each other. Alternatively, the computing device 100 may be a medical robot that supports or assists the overall medical practice performed at medical sites. In this case, the medical robot may include a venipuncture device that includes a blood collection or intravenous injection (IV) function for diagnosing disease, a blood transfusion, and/or the like. Since the above description is only one example related to the type of computing device 100, the type of computing device 100 may be configured in various manners within a range understandable to those skilled in the art based on the content of the present disclosure. Since the above description is only one example related to the type of computing device 100, the type of computing device 100 may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 shows only an example, and thus, the computing device 100 may include other components for implementing a computing environment. Furthermore, only some of the components disclosed above may be included in the computing device 100.

The processor 110 according to one embodiment of the present disclosure may be understood as a constituent unit including hardware and/or software for performing computing operations. For example, the processor 110 may read a computer program and perform data processing for machine learning. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

According to the present disclosure, the processor 110 may generate condition change data for controlling a medical system based on the multi-modal data generated from the medical system. The processor 110 may train a first artificial intelligence model, and may generate condition change data by using the trained first artificial intelligence model. To obtain high-quality multi-modal data, the processor 110 may calculate the suitability of the multi-modal data, and may change the control conditions of the medical system and obtain multi-modal data again when the multi-modal data has a suitability lower than a reference value.

In addition, the processor 110 may cause a medical operation to be performed in the medical system by using multi-modal data having a suitability equal to or higher than the reference value. In this case, the processor 110 may output the reliability of the medical operation by inputting the multi-modal data to a trained second artificial intelligence model. When the reliability of the medical operation is equal to or higher than a reference value, the processor 110 may cause a medical operation to be performed in the medical system, and may generate condition change data by using the multi-modal data when the reliability of the medical operation is lower than the reference value.

The processor 110 may train the first and second artificial intelligence models through supervised learning using training data as input values. Alternatively, the first and second artificial intelligence models may be trained through unsupervised learning in which criteria for data recognition are discovered by learning the types of data required for data recognition on their own without any special guidance. Alternatively, the first and second artificial intelligence models may be trained through reinforcement learning in which there is utilized feedback on whether the results of data recognition according to learning are correct.

The first and second artificial intelligence models may each include at least one neural network. The neural network may include, but is not limited to, network models such as a Deep Neural Network (DNN), a Recurrent Neural Network (RNN), a Bidirectional Recurrent Deep Neural Network (BRDNN), Multilayer Perceptron (MLP), and a Convolutional Neural Network (CNN).

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data required for the processor 110 to perform operations, combinations of data, and program codes executable by the processor 110. Furthermore, the memory 120 may store the program codes that cause the processor 110 to generate training data.

The memory 120 may store the multi-modal data generated from a medical system, and may also store the condition change data generated based on the data.

The network unit 130 according to one embodiment of the present disclosure may be understood as a constituent unit that transmits and receives data through any type of known wired/wireless communication system. For example, the network unit 130 may perform data transmission and reception by using a wired/wireless communication system such as a local area network (LAN), a wideband code division multiple access (WCDMA) network, a long term evolution (LTE) network, the wireless broadband Internet (WiBro), a 5th generation mobile communication (5G) network, an ultra-wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data, required for the processor 110 to perform operations, through wired or wireless communication with any system, any client, or the like. Furthermore, the network unit 130 may transmit data, generated through the operations of the processor 110, through wired or wireless communication with any system, any client, or the like. For example, the network unit 130 may receive multi-modal data through communication with a picture archiving and communication system, a cloud server for performing tasks such as the standardization of medical data, a medical robot, or the like. The network unit 130 may transmit various types of data, generated through the operations of the processor 110, through communication with the above-described system, server, or medical robot, or the like.

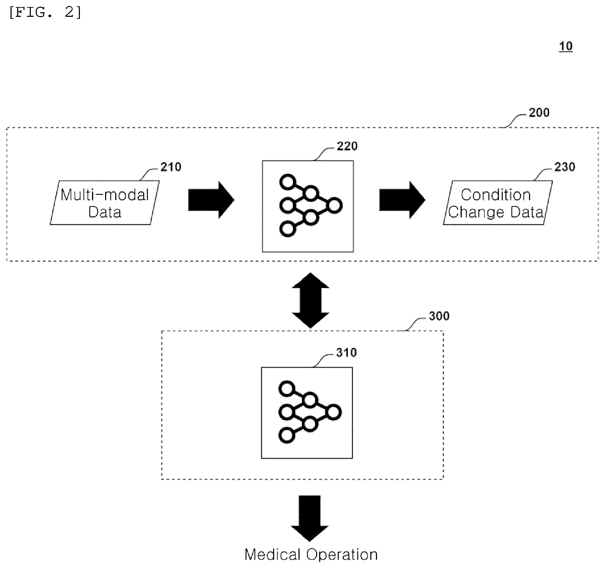

FIG. 2 is a block diagram of a medical system control apparatus according to one embodiment of the present disclosure.

Referring to FIG. 2, the computing device of FIG. 1 may be configured to include a medical system control apparatus 200 and the second artificial intelligence model 310 of a medical system 300. In FIG. 2, the first artificial intelligence model 220 of the medical system control apparatus 200 and the second artificial intelligence model 310 of the medical system 300 are depicted separately, but the individual models may be integrated into a single model.

The medical system control apparatus 200 may receive multi-modal data including data of various dimensions from the medical system 300, and may generate condition change data 230 based on the multi-modal data. The medical system control apparatus 200 may generate condition change data 230 by inputting the multi-modal data 210 to the trained first artificial intelligence model 220.

The multi-modal data 210 may include a medical image, and the medical image may be a two-dimensional image or a three-dimensional image in which two-dimensional images are stacked in multiple layers.

The multi-modal data 210 may include sensing values obtained from various sensors included in the medical system 300. For example, the sensing values may be obtained from various sensors such as a distance sensor such as an ultrasonic sensor or laser sensor, a pressure sensor, a gyro sensor, and an acceleration sensor.

The multi-modal data 210 may include control parameters for various motors included in the medical system 300. For example, when the medical system 300 includes a medical robot, the control parameters may include encoder values of the motors for the individual axes of the robot. Meanwhile, the multi-modal data 210 may include not only the raw data obtained from the medical system 300, but also the values obtained through analysis using the raw data.

The first artificial intelligence model 220 may receive multi-modal data 210 of various dimensions and infer condition change data 230. For example, individual types of data included in the multi-modal data 210 have different characteristic dimensions, so that the first artificial intelligence model 220 may unify the multi-modal data 210 into data having the same characteristic dimensions. Alternatively, the first artificial intelligence model 220 may include individual models corresponding to respective types of data included in the multi-modal data 210. Each model of the first artificial intelligence model 220 separately performs inference for each type of data of the multi-modal data 210, and the first artificial intelligence model 220 may integrate individual inference values. In this case, the first artificial intelligence model 220 may assign different weights to the individual inference values. The inference method of the first artificial intelligence model 220 is not limited thereto.

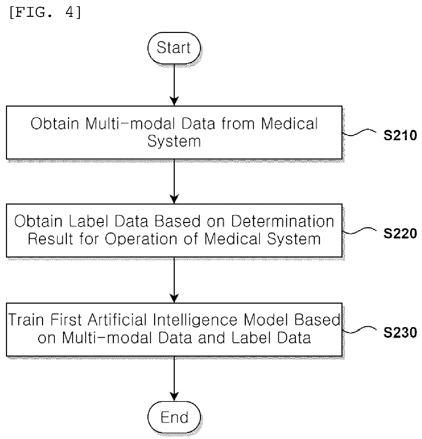

A method of training the first artificial intelligence model 220 will be described later with reference to FIG. 4.

The medical system control apparatus 200 may generate condition change data 230 for changing control conditions of the medical system 300. The condition change data 230 includes information for changing control conditions of various components included in the medical system 300. For example, the condition change data 230 may control the operation of a sensor or motor of the medical system 300.

The medical system control apparatus 200 may generate the condition change data 230 for controlling the medical system 300 by using the multi-modal data 210 generated in the medical system 300. That is, the medical system control apparatus 200 may provide feedback on the medical system 300. Accordingly, the quality and accuracy of various types of data generated in the medical system 300 and the operation of the medical system 300 may be improved.

As an embodiment, the medical system 300 may include a medical image acquisition device. For example, when the medical image acquisition device acquires an ultrasound image, the medical system 300 may include an ultrasound probe and a sensor for measuring the distance to a body part. In this case, the multi-modal data 210 may include an ultrasound image, the distance between the ultrasound probe and the body, the contact pressure between the ultrasound probe and the body, the displacement and location of the ultrasound probe, and the time at which the ultrasound image is output. Furthermore, the multi-modal data 210 may include the curvature information of a measurement site (e.g., an upper arm) generated based on the distance measured by the medical system 300.

The medical system control apparatus 200 may generate the condition change data 230 that controls the medical system 300 so that the contact pressure falls within a reference range. Based on the condition change data 230 generated in this manner, the medical system 300 may position the ultrasound probe in a range where a blood vessel is not compressed.

As an embodiment, the medical system 300 may further include a body compression unit for compressing a body part. The body compression unit may compress, for example, an upper arm. The body compression unit may include a cuff. The body compression unit may perform an operation for compressing a body part together with components such as an ultrasound probe, an invasive vascular fixation unit, and a local vascular compression unit.

The multi-modal data 210 may include an ultrasound image, the degree of body compression and compression time of the body compression unit, and ultrasound image acquisition time. The medical system control apparatus 200 may analyze the characteristics of the blood vessel area of an ultrasound image, and may detect blood vessels such as arteries and veins in the ultrasound image based on the characteristics of the blood vessel area and the control parameters of the ultrasound probe. The medical system control apparatus 200 may generate the condition change data 230 that controls the medical system 300 to facilitate blood vessel detection in an ultrasound image. Based on the condition change data 230 generated in this manner, the medical system 300 may obtain an optimal ultrasound image that allows blood vessel detection in a range where a blood vessel is not compressed.

As an embodiment, the medical system 300 may include a venipuncture device. Although the venipuncture device is described as an example of a blood collection device in the following description, the following description may be similarly applied to a venous injection device. The venipuncture device may include an injection unit for performing blood collection, a driving unit for driving the injection unit, a motor unit, a sensor unit, and a search unit. In this case, the multi-modal data 210 may include an image of a blood collection site acquired through the search unit, the distance between the injection unit and the body, the encoder value of the motor unit, the sensing value of the sensor unit, and the location of the venipuncture device. The image of the blood collection site may be an ultrasound image or an infrared image. The location of the venipuncture device may mean the physical location of at least one of the injection unit, the driving unit, the motor unit, the sensor unit, and the search unit. For example, it may mean the location of a needle or needle driving unit included in the injection unit, the location of an ultrasound probe included in the search unit, or the like.

The medical system control apparatus 200 may determine a blood vessel area to be a blood collection target based on an image, and may generate condition change data 230 to position the blood collection device in the blood vessel area. Based on the condition change data 230 generated in this manner, the medical system 300 may safely perform a blood collection operation at an optimal location.

The medical system 300 may perform a medical operation based on the multi-modal data 210 by using the trained second artificial intelligence model 310. In this case, the second artificial intelligence model 310 may output the reliability of the medical operation. The medical system 300 may compare the reliability with a preset reference value, and may perform the medical operation or obtain condition change data 230 again based on the result of the comparison.

The medical operation may include an operation such as the operation of detecting a blood vessel from a medical image, the operation of performing a blood collection operation, or the operation of diagnosing a lesion included in a medical image.

As an embodiment, the second artificial intelligence model 310 may infer features of a blood vessel area of an ultrasound image between the ultrasound probe and the body based on control parameters of the ultrasound probe and the contact pressure between the ultrasound probe and the body included in the multi-modal data.

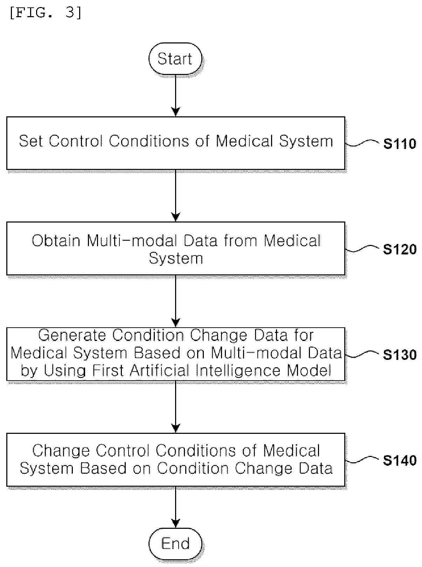

FIG. 3 is a flowchart showing a medical system control method according to one embodiment of the present disclosure.

Referring to FIG. 3, control conditions of a medical system may be set in step S110. For example, the medical system 300 may include a medical image acquisition device or a venipuncture device.

The medical system control apparatus 200 may obtain multi-modal data from the medical system in step S120. For example, the medical system 300 may include an ultrasound probe, and the multi-modal data 210 may include an ultrasound image.

The medical system control apparatus 200 may generate condition change data for changing the set control conditions of the medical system based on the multi-modal data by using the first artificial intelligence model in step S130.

For example, when the medical system 300 includes an ultrasound medical image acquisition device, the medical system control apparatus 200 may generate condition change data to change at least one of the position of an ultrasound probe and the contact pressure between the ultrasound probe and the body.

For example, when the medical system 300 includes a venipuncture device, the medical system control apparatus 200 may generate condition change data to change at least one of the degree of body compression of the body compression unit included in the venipuncture device and the location of the venipuncture device. The location of the venipuncture device may mean the physical location of at least one of an injection unit, a driving unit, a motor unit, a sensor unit, and a search unit.

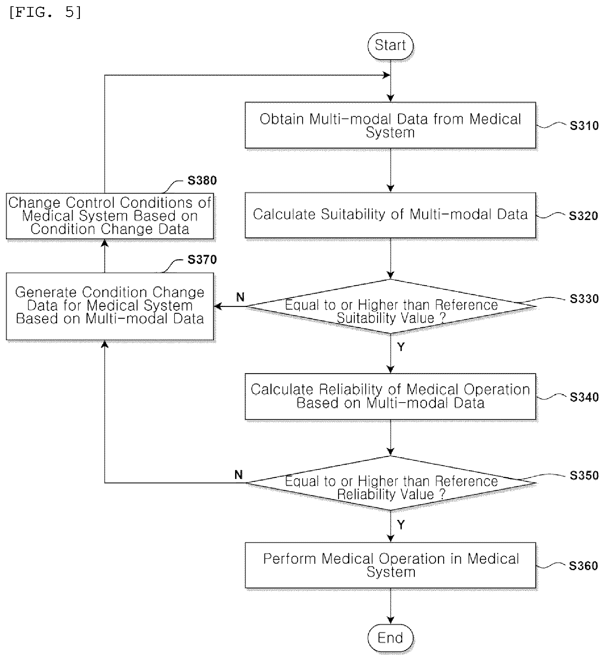

Meanwhile, whether to perform step S130 may be determined based on the suitability of the multi-modal data 210. This will be described later with reference to FIG. 5.

The control conditions of the medical system may be changed based on the condition change data in step S140.

FIG. 4 is a flowchart showing a method of training the first artificial intelligence model according to one embodiment of the present disclosure.

Referring to FIG. 4, the medical system control apparatus 200 may obtain multi-modal data from a medical system in step S210.

The medical system control apparatus 200 may obtain label data based on the result of determination for the operation of the medical system in step S220. The medical system control apparatus 200 may obtain the label data generated by medical staff (supervised learning) through communication with an external device (a server or the like), or may generate label data based on medical staff's commands (semi-supervised learning). Alternatively, the medical system control apparatus 200 may generate label data within itself on its own (unsupervised learning).

For example, the medical system may perform a preset operation while obtaining multi-modal data, and the result of the operation may be quantitatively determined.

As an embodiment, the medical system may perform an operation of capturing a medical image. In this case, the medical staff may quantitatively determine the quality of the captured medical image. Alternatively, a patient's condition may be monitored in the situation in which a medical image is being captured. The medical system control apparatus 200 may obtain label data based on these results.

As an embodiment, the medical system 300 may perform a blood collection operation for a patient. In this case, the medical system 300 may press the patient's body part for blood collection and insert a needle. In this case, the medical system control apparatus 200 may obtain label data based on the degree of pressing felt by the patient, the degree of smooth blood collection, and the safety of a blood collection path.

Meanwhile, the operation of the medical system is not limited thereto, and may perform an operation such as the operation of detecting a lesion or the operation of administering intravenous injection based on an medical image.

The medical system control apparatus 200 may train the first artificial intelligence model based on the multi-modal data and the label data in step S230.

FIG. 5 is a flowchart showing a medical system control method to which condition control is applied according to one embodiment of the present disclosure.

Referring to FIG. 5, the computing device may obtain multi-modal data from a medical system in step S310.

The computing device may calculate the suitability of the obtained multi-modal data in step S320. In the present specification, the suitability means that the quality or quantity of the multi-modal data 210 is sufficiently secured for the medical system to perform a preset operation based on the multi-modal data 210. For example, when the medical system 300 performs image-based lesion detection, the suitability of the multi-modal data 210 may mean the quality of an image. For example, when the medical system 300 performs a blood collection operation, the suitability of the multi-modal data 210 may mean a determination criterion indicating whether blood vessel detection from an image is easy.

As an embodiment, the computing device may include a separate computational model for determining the suitability of multi-modal data. The computational model may be implemented as a rule-based mathematical algorithm or an artificial intelligence model, but is not limited thereto.

The computing device determines whether the suitability is equal to or higher than a reference value in step S330. In this case, the medical system control apparatus 200 may determine the suitability for one piece of multi-modal data, and may determine the suitability for multiple multi-modal data 210 accumulated over a predetermined period. That is, the medical system control apparatus 200 may determine that the suitability is equal to or higher than the reference value when multi-modal data 210 having a suitability equal to or higher than the reference value is input for a predetermined period.

When the suitability is equal to or higher than the reference value, the computing device may calculate the reliability of the medical operation based on the multi-modal data in step S340. For example, the computing device may infer the reliability of the medical operation by using the second artificial intelligence model.

The computing device determines whether the reliability of the medical operation is equal to or higher than a preset reference value in step S350. When the reliability is equal to or higher than the reference value, the computing device may cause the medical system to perform a preset operation in step S360. For example, the preset operation may include at least one of a medical imaging operation, a blood collection operation, an operation of detecting a lesion in a medical image, an operation of controlling a medical robot, and an operation of observing a body organ based on a medical robot.

When the suitability of the multi-modal data is lower than the reference value in step S330 or when the reliability of the medical operation is lower than the reference value in step S350, the computing device may generate condition change data for the medical system based on the multi-modal data in step S370. For example, the computing device may generate condition change data by using the multi-modal data and the first artificial intelligence model. In this case, the condition change data 230 may be generated in a direction in which the medical system 300 is controlled to obtain the multi-modal data 210 having a higher suitability. The computing device may change the control conditions of the medical system based on the condition change data in step S380.

Meanwhile, when the suitability of the multi-modal data is lower than the reference value in step S330, the control conditions related to components for generating and obtaining the multi-modal data within the medical system may be changed through steps S370 and S380. For example, the condition change data may be generated to change the control-related parameters of a body compression unit included in a medical robot, the control-related parameters of an ultrasound probe included in a medical image acquisition device, or the like.

In addition, when the reliability of the medical operation is lower than the reference value in step S350, the control conditions related to the components for performing the medical operation within the medical system may be changed through steps S370 and S380. For example, the condition change data may be generated to change parameters related to the blood collection operation of a medical robot, parameters applied to a blood vessel detection algorithm from a medical image, or parameters applied to a lesion diagnosis algorithm from a medical image.

The above description of the present disclosure is intended for illustrative purposes, and those having ordinary skill in the art to which the present disclosure pertains will understand that it may be easily modified into other specific forms without departing from the technical spirit or essential features of the present disclosure. Therefore, it should be understood that the embodiments described above are illustrative but not limitative in all respects. For example, each component described as a single type may be implemented in a distributed form, and likewise, components described as distributed may be implemented in a combined form.

The scope of the present disclosure is defined by the claims described below rather than the detailed description above, and all changes or modifications derived from the meanings and scope of the claims and their equivalent concepts should be interpreted as being included in the scope of the present disclosure.

## Claims

1. A medical system control method, the medical system control method being performed by a computing device including at least one processor, the medical system control method comprising:
obtaining multi-modal data from a medical system that operates according to set control conditions;
generating condition change data for changing the control conditions of the medical system based on the multi-modal data by using a first artificial intelligence model; and
controlling the medical system based on the condition change data.

2. The medical system control method of claim 1, wherein the generating comprises:
calculating suitability of the multi-modal data; and
generating the condition change data when the suitability is lower than a reference value.

3. The medical system control method of claim 2, further comprising:
performing a preset medical operation by using the medical system when the suitability is equal to or higher than the reference value.

4. The medical system control method of claim 3, wherein the performing a preset medical operation comprises:
calculating reliability of the medical operation based on the multi-modal data by using a second artificial intelligence model;
performing the medical operation when the reliability is equal to or higher than a reference value; and
generating the condition change data when the reliability is lower than the reference value.

5. The medical system control method of claim 4, wherein:
the medical system comprises an ultrasound probe; and
the multi-modal data comprises an ultrasound image.

6. The medical system control method of claim 5, wherein the condition change data changes at least one of a position of the ultrasound probe and a contact pressure between the ultrasound probe and a body.

7. The medical system control method of claim 6, wherein the second artificial intelligence model is a model trained to infer characteristics of a blood vessel area of the ultrasound image based on the contact pressure between the ultrasound probe and the body included in the multi-modal data.

8. The medical system control method of claim 4, wherein:
the medical system comprises a venipuncture device; and
the multi-modal data includes an infrared image or an ultrasound image.

9. The medical system control method of claim 8, wherein the generating comprises:
generating the condition change data to change at least one of a degree of body compression of a body compression unit included in the venipuncture device and a position of the venipuncture device.

10. A medical system control apparatus, comprising:
memory configured to store multi-modal data obtained from a medical system; and
a processor configured to calculate suitability of the multi-modal data, to generate condition change data for changing control conditions of the medical system based on the multi-modal data by using a first artificial intelligence model when the suitability is lower than a reference value, and to perform a preset medical operation by using the medical system when the suitability is equal to or higher than the reference value.

11. The medical system control apparatus of claim 10, wherein the medical system comprises a medical image acquisition device or a venipuncture device.

12. The medical system control apparatus of claim 11, wherein the multi-modal data:
comprises a medical image; and
further comprises at least one of a time at which the medical image is acquired, a sensing value obtained by a sensor included in the medical image acquisition device or the venipuncture device, an encoder value of a motor included in the medical image acquisition device or the venipuncture device, and a position, displacement, and operation time of an ultrasound probe included in the medical image acquisition device or the venipuncture device.

13. A computer program stored in a computer-readable storage medium, the computer program causing operations for controlling a medical system to be performed when executed by at least one processor, wherein the operations comprise operations of:
obtaining multi-modal data from a medical system that operates according to set control conditions;
calculating suitability of the multi-modal data; and
generating condition change data for changing the control conditions of the medical system based on the multi-modal data by using a first artificial intelligence model when the suitability is lower than a reference value, and performing a preset medical operation by using the medical system when the suitability is equal to or higher than the reference value.
